# EUROPÄISCHE PATENTSCHRIFT

(11) **EP 0 531 980 B1**
(45) Veröffentlichungstag und Bekanntmachung des Hinweises auf die Patenterteilung: **17.05.1995**
(21) Anmeldenummer: 92115438.1
(22) Anmeldetag: 09.09.1992
(51) Int. Cl.: B05B 7/00, B05B 17/06, A01M 7/00

(54) **Vorrichtung zum Zerstäuben eines Wirkstoffs**
Device for spraying an active substance
Dispositif de pulvérisation d'une substance active

(30) Priorität: 10.09.1991 DE 9111204 U
(43) Veröffentlichungstag der Anmeldung: 17.03.1993
(73) Patentinhaber: Stahl, Werner, D-88662 Überlingen (DE)
(72) Erfinder: Stahl, Werner, D-88662 Überlingen (DE)
(74) Vertreter: Fincke, Karl Theodor, Dipl.-Phys. Dr.

(56) Entgegenhaltungen:
- EP-A- 0 081 261
- FR-A- 2 285 930
- US-A- 3 164 324
- PATENT ABSTRACTS OF JAPAN vol. 005, no. 120 (C-065)4. August 1981 & JP-A-56 058 555 (TERAOKA CHITOSHI) 21. MAI 1981

## Beschreibung

Die Erfindung betrifft eine Vorrichtung zum Zerstäuben eines Wirkstoffs - insbesondere zur Schädlingsbekämpfung, zum Pflanzenschutz, zum Vorratsschutz, zur Desinfektion, zur Geruchsneutralisation oder zur Entkeimung - mit einem einen Luftführungs-Außenmantel umschließenden Gehäuse, mit einem in einem Einlaßabschnitt des Luftführungs-Außenmantels angeordneten Ventilator, mit einem stromabwärts des Ventilators angeordneten Luftführungs-Innenmantel, mit sich zwischen dem Luftführungs-Außenmantel und dem Luftführungs-Innenmantel erstreckenden Luftleitprofilen und mit einer von dem Luftführungs-Innenmantel umschlossenen Zerstäuberanordnung zum Zerstäuben von ihr zugeführtem, aus einer stromabwärts gerichteten Wirkstoffaustrittsöffnung im Luftführungs-Innenmantel austretendem Wirkstoff.

Bei einer nach dem Prospekt "turbosonic" der Anmelderin bekannten Vorrichtung dieser Art befindet sich in einem von dem Luftführungs-Innenmantel umschlossenen Gehäuse eine Ultraschall-Zerstäuberanordnung. Von dieser Ultraschall-Zerstäuberanordnung nicht zerstäubter Wirkstoff sammelt sich in einem Sumpf des Gehäuses und muß daher mit einer Absaugeinrichtung abgesaugt werden.

Nach einem Prospekt "Green House Sprayer" ist eine ähnliche Vorrichtung bekannt, die mit einer Kompressor-Zerstäuberanordnung versehen ist. Eine solche Vorrichtung ist voluminös, außerordentlich schwer und muß auf Rädern montiert werden.

Gleiches gilt für eine ähnliche Vorrichtung nach einem Prospekt "Fontan Turbostar-E" der Firma Motan. (Alle diese Prospekte sind als Anlage beigefügt.)

Eine ähnliche Vorrichtung ist in Dokument US-A-3164324 beschrieben.

Aufgabe der Erfindung ist es, eine Vorrichtung nach dem Oberbegriff des Anspruchs 1 anzugeben, die bei leichter, nicht voluminöser Bauweise keine Absaugung von nicht zerstäubtem Wirkstoff erfordert.

Zur Lösung dieser Aufgabe ist die Vorrichtung dadurch gekennzeichnet, daß sich in dem Luftführungs-Innenmantel ein Kompressor befindet, der Luft durch einen die Wirkstoffaustrittsöffnung umschließenden Ringeinlaß und einen zwischen dem Kompressor und dem Luftführungs-Innenmantel gebildeten Ringkanal in seine dem Ventilator zugewandte Einlaßöffnung einsaugt und die von ihm komprimierte Luft durch seine der Wirkstoffaustrittsöffnung zugewandte Auslaßöffnung einer die Wirkstoffaustrittsöffnung umschließenden Wirbelstromdüse zuführt.

Bei der erfindungsgemäßen Vorrichtung kann wegen der Wirbelstromdüse der Kompressor verhältnismäßig klein und leicht sein. Beispielsweise reicht ein Staubsauger-Kompressor aus, der 40 bis 70 l Luft pro Sekunde mit einem Druck von 120 bis 140 mbar bei einer Leistungsaufnahme von 1000 bis 1200 Watt abgibt. Ist die Zerstäuberanordnung eine Ultraschall-Zerstäuberanordnung, so verhindert die Wirbelstromdüse ein Abtropfen von Wirkstoff beim Stoppen der Wirkstoffzerstäubung.

Eine besonders einfach aufgebaute und für die Zwecke der Erfindung wirkungsvolle Wirbelstromdüse weist einen Kranz von unter gleichen Winkeln zu Radialen geneigten, einen zentralen Bereich frei lassenden Leitflächen auf, deren stromabwärtige Kanten radial außen durch eine Ringfläche abgedeckt sind. Durch die Ringfläche wird der der Wirbelstromdüse zugeführte Luftstrom radial nach innen umgelenkt und erhält durch die Leitflächen einen Wirbel mit zur Wirbelstromdüse koaxialer Achse. Dabei sind in Anpassung an die vorliegenden Verhältnisse die Leitflächen bevorzugt eben oder gekrümmt.

Um den Wirkstoff innerhalb der Wirbelstromdüse zu zerstäuben, ist bevorzugt an die stromaufwärtigen Kanten der Wirbelstromdüse ein Körper einer Wirkstoff-Zuführungsdüse angesetzt, die einen in den zentralen Bereich der Wirbelstromdüse ragenden Wirkstoff-Austrittsnippel aufweist.

Bevorzugt ist an die Wirkstoff-Zuführungsdüse ein Ultraschallgeber gekoppelt, der die Wirkstoff-Zuführungsdüse in Axialschwingungen versetzt, und es ist das Austrittsende des Austrittsnippels der Wirkstoff-Zuführungsdüse von einem Radialflansch umschlossen. Der Wirkstoff strömt dann aus dem Austrittsnippel auf die stromabwärtsseitige Fläche des Radialflansches, bildet dort wegen der Axialschwingungen Wellen, und von den Kuppen der Wellen lösen sich sehr feine Wirkstofftröpfchen ab, beispielsweise mit einem Tröpfchendurchmesser-Spektrum, dessen Maximum je nach der verwendeten Erregerfrequenz bei weniger als 50 »m liegt, z. B. bei einer Erregerfrequenz von 120 KHz um 12 »m liegt. Der Wirbelstrom aus der Wirbelstromdüse verhindert dabei nicht nur beim Stoppen des Ultraschallgebers oder der Wirkstoffzufuhr ein Abtropfen von Wirkstoff von der Wirkstoff-Zuführungsdüse, sondern auch eine Überlastung der stromabwärtsseitigen Fläche des Ringflansches mit einem zu dicken Wirkstoffilm. Unabhängig hiervon ermöglicht die Kombination von Wirbelstrom- und Ultraschallzerstäubung eine Erhöhung des Luftstroms durch die Vorrichtung und damit der Wurfweite der Vorrichtung, eine noch feinere Zerstäubung als mit einer dieser Zerstäubungsarten allein und ein feines homogenes Zerstäuben auch relativ hochviskoser Wirkstoffe, Wirkstoffsuspensionen und Wirkstoffemulsionen, das mit einer der Zerstäubungsarten allein nicht möglich wäre. Auch ist mittels der Wirbelstromdüse die stromabwärtsseitige Fläche des Radialflanschs einfach und wirksam zu reinigen.

Besonders wirksam und von geringem Gewicht ist ein zwischen einem Halteflansch der Wirkstoff-Zuführungsdüse und einem Wirkstoffeinlaß der Wirkstoff-Zuführungsdüse eingesetzter ringförmiger Piezoerreger.

Damit der Piezoerreger dem Radialflansch am Austrittsende des Austrittsnippels eine hohe Schwingungsamplitude erteilt, ist bevorzugt an die dem Wirkstoffeinlaß der Wirkstoff-Zuführungsdüse zugewandte Seite des Piezoerregers ein Ringblock angesetzt, der eine wesentlich größere Masse hat als der stromabwärts des Halteflansches liegende Teil der Wirkstoff-Zuführungsdüse.

Um zu verhindern, daß in den Kompressor Umgebungsschmutz gerät, ist der oben genannte Ringeinlaß bevorzugt durch ein Ringfilter abgedeckt.

Um zu dem Kompressor und gegebenenfalls zu dem Ultraschallgeber elektrische Leitungen zu führen, ist bevorzugt wenigstens eines der oben genannten Luftleitprofile hohl und nimmt diese elektrischen Leitungen auf.

Um wahlweise Spülmittel oder Wirkstoff der Wirkstoff-Zuführungsdüse zuführen zu können, sind bevorzugt von einem Spülmitteltank und einem Wirkstofftank im Gehäuse und gegebenenfalls von einem äußeren an die Vorrichtung anzuschließenden dritten Tank Leitungen zu einer gemeinsamen, eine Pumpe enthaltenden Zuführungsleitung zu der Wirkstoff-Zuführungsdüse geführt.

Ist der Wirkstoff im Wirkstofftank eine Suspension, so ist bevorzugt dem Wirkstofftank eine eine zweite Pumpe enthaltende Umwälzleitung zugeordnet, mittels der die Suspension im Wirkstofftank homogen zu halten ist.

Um die Vorrichtung nach Einschalten ohne weitere Bedienung arbeiten lassen zu können, befinden sich bevorzugt in den Leitungen von einem Programmsteuerungs- und einem Ja/Nein-Durchflußmesser in der gemeinsamen Zuführungsleitung steuerbare Magnetventile. Stellt beispielsweise der Durchflußmesser fest, daß kein Wirkstoff mehr fließt, weil etwa der Wirkstofftank leer ist, so bewirkt er eine selbsttätige Umschaltung auf den Spülmitteltank.

Ein besonders rationeller Aufbau ergibt sich dann, wenn im Raum zwischen dem Gehäuse und dem Luftführungs-Außenmantel oben symmetrisch zur vertikalen Längsmittelebene des Gehäuses je ein Tank angeordnet ist und in diesem Raum unten Elektroinstallationen vorgesehen sind.

Die Erfindung wird im folgenden an Ausführungsbeispielen unter Hinweis auf die beigefügten Zeichnungen beschrieben.
- Fig. 1: zeigt einen Axialschnitt durch eine Ausführungsform der Vorrichtung.

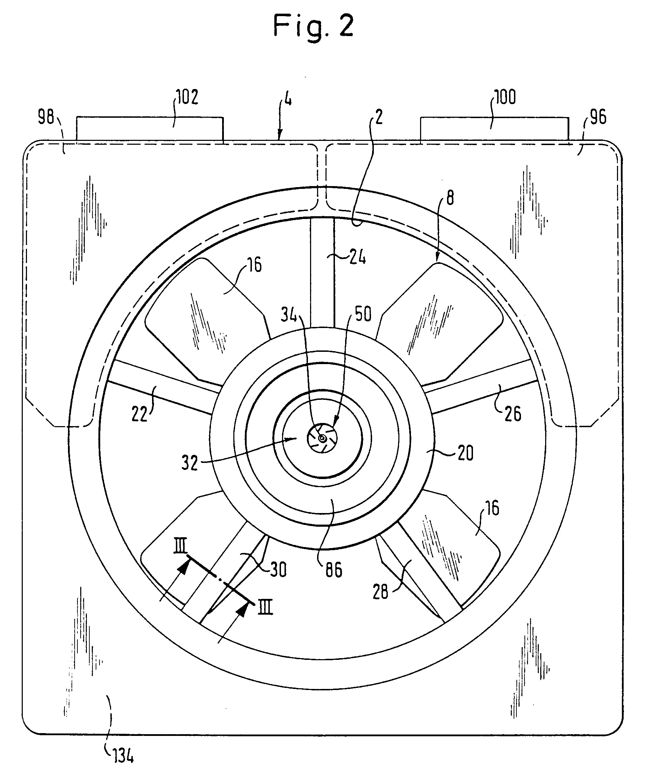

- Fig. 2: zeigt eine Ansicht der Vorrichtung nach Fig. 1 aus der Blickrichtung II in Fig. 1.
- Fig. 3: zeigt einen Schnitt längs der Linie III-III in fig. 2.
- Fig. 4: zeigt vergrößert den Bereich IV in Fig. 1 mit einer Wirbelstromdüse.
- Fig. 5: zeigt die Wirbelstromdüse nach Fig. 4 aus der Blickrichtung V in Fig. 4.
- Fig. 6: zeigt in einer der Fig. 5 entsprechenden Ansicht eine anders ausgebildete Wirbelstromdüse.
- Fig. 7: zeigt in einer der Fig. 4 entsprechenden Ansicht eine mit einem Ultraschallgeber vereinigte Wirbelstromdüse.
- Fig. 8: zeigt ein Schaltschema der Vorrichtung.

Die Vorrichtung nach dem Ausführungsbeispiel weist ein einen Luftführungs-Außenmantel 2 umschließendes Gehäuse 4 auf. In einem Einlaßabschnitt 6 des Luftführungs-Außenmantels 2 ist ein Ventilator 8 angeordnet. Träger des Ventilators 8 ist ein auch als Schutz dienender Stern 10, dessen Enden an einer Stirnfläche 12 des Gehäuses 4 befestigt sind. Der Ventilator 8 weist einen inneren Stator (nicht sichtbar) und einen Rotor 14 auf, der an seinem Umfang einen Kranz von Ventilatorflügeln 16 trägt. Durch den Stern 10 wird von dem Ventilator 8 Luft angesaugt, in den von dem Luftführungs-Außenmantel 2 umschlossenen Raum 18 gefördert und tritt aus diesem Raum 18 axial aus dem Gehäuse 4 aus. Stromabwärts des Ventilators 8 ist innerhalb des Luftführungs-Außenmantels 2 ein Luftführungs-Innenmantel 20 angeordnet, der den Raum 18 zu einem Ringraum macht. Zwischen dem Luftführungs-Außenmantel 2 und dem Luftführungs-Innenmantel 20 erstrecken sich Luftleitprofile 22, 24, 26, 28, 30, die den von dem Ventilator 8 erzeugten Luftstrom laminarisieren und überdies den Luftführungs-Innenmantel 20 zentrisch im Luftführungs-Außenmantel 2 halten.

Das stromabwärtige Ende des Luftführungs-Innenmantels 20 umschließt eine Zerstäuberanordnung 32 zum Zerstäuben von ihr zugeführtem, aus einer stromabwärts gerichteten Wirkstoff-Austrittsöffnung 34 im Luftführungs-Innenmantel 20 austretendem Wirkstoff.

In dem Luftführungs-Innenmantel 20 ist mittels Schrauben 36, die Distanzbuchsen 38 durchsetzen, zentrisch ein Kompressor 40 gehaltert, der Luft durch einen die Wirkstoff-Austrittsöffnung 34 umschließenden Ringeinlaß 42 und einen zwischen dem Kompressor 40 und dem Luftführungs-Innenmantel 20 gebildeten Ringkanal 44 in seine dem Ventilator 8 zugewandte Einlaßöffnung 46 einsaugt und die von ihm komprimierte Luft durch seine der Wirkstoff-Austrittsöffnung 34 zugewandte Auslaßöffnung 48 einer die Wirkstoff-Austrittsöffnung 34 umschließenden Wirbelstromdüse 50 zuführt.

Aus der Wirbelstromdüse 50 tritt zerstäubter Wirkstoff in Form eines Strahls aus, der von einem im wesentlichen laminaren Mantelluftstrom aus dem Raum 18 umgeben ist. Durch Verändern der Drehzahl des Ventilators 8 läßt sich die Reichweite des Gesamtstrahls, der aus der Vorrichtung austritt, einstellen.

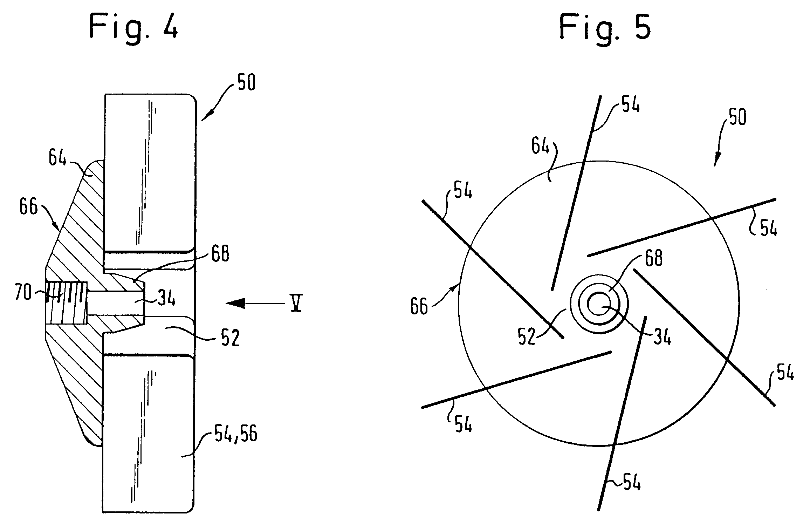

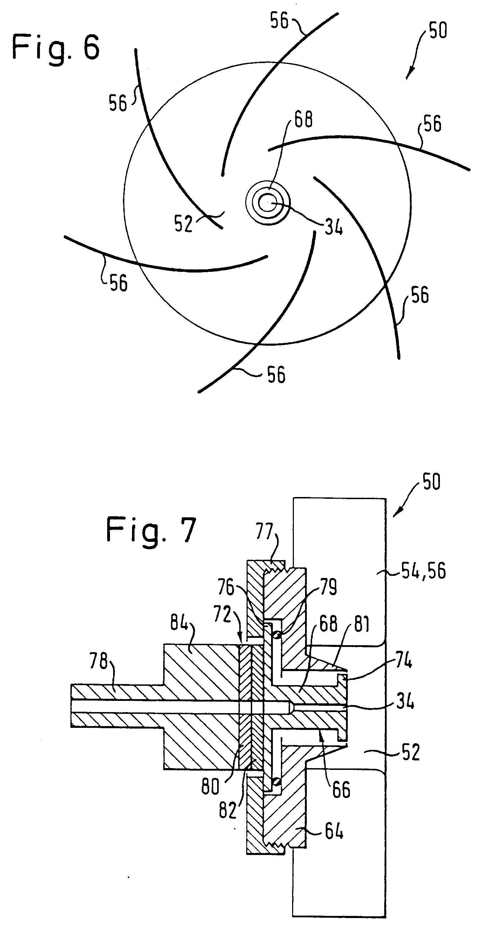

Die Wirbelstromdüse 50 weist einen Kranz von unter gleichen Winkeln zu Radialen geneigten, einen zentralen Bereich 52 frei lassenden Leitflächen 54 bzw. 56 auf, deren stromabwärtige Kanten radial außen durch eine Ringfläche an einer Ringkappe 58 abgedeckt sind, die zum Haltern der Wirbelstromdüse 50 an einem Austrittsstutzen 60 des Kompressors 40 dient. In dem Austrittsstutzen 60 befinden sich Widerlageransätze 62 für die Wirbelstromdüse 50. Bei der Ausführungsform nach Fig. 5 sind die Leitflächen 54 eben, bei der Ausführungsform nach Fig. 6 sind die Leitflächen 56 gekrümmt.

An die stromaufwärtigen Kanten der Wirbelstromdüse 50 ist ein Körper 64 einer Wirkstoff-Zuführungsdüse 66 angesetzt, die einen in den zentralen Bereich 52 der Wirbelstromdüse 50 ragenden Wirkstoff-Austrittsnippel 68 und einlaßseitig ein Innengewinde 70 zum Anschluß einer Wirkstoff-Zuführungsleitung aufweist.

Bei der Ausführungsform nach Fig. 7 ist an die Wirkstoff-Zuführungsdüse 66 ein Ultraschallgeber 72 gekoppelt, der die Wirkstoff-Zuführungsdüse 66 in Axialschwingungen versetzt. Das Austrittsende ihres Wirkstoff-Austrittsnippels 68 ist von einem Radialflansch 74 umschlossen, der die eingangs genannte Bedeutung hat. Als Ultraschallgeber 72 ist zwischen einem Halteflansch 76 der Wirkstoff-Zuführungsdüse 66 und einem Wirkstoff-Einlaßstutzen 78 der Wirkstoff-Zuführungsdüse 66 ein zwei Ringe 80, 82 aufweisender Piezoerreger eingesetzt. An die dem Wirkstoffeinlaßstutzen 78 zugewandte Seite des Piezoerregers 80, 82 ist ein Ringblock 84 angesetzt, der eine wesentlich größere Masse hat als der stromabwärts des Halteflansches 76 liegende Teil der Wirkstoff-Zuführungsdüse 66. Die Erregungsfrequenz des Piezoerregers 80, 82 ist auf eine Resonanzfrequenz der Wirkstoff-Zuführungsdüse 66 und der mit ihr verbundenen Massen eingestellt. Der Halteflansch 76 ist mittels einer Überwurfmutter 77 unter Zwischenfügung eines elastischen Rings 79 an den Körper 64 geschraubt. Da der Körper 64 über die Leitflächen 54 bzw. 56, den Austrittsstutzen 60, den Kompressor 40, die Schrauben 36, die Distanzbuchsen 38, den Luftführungs-Innenmantel 20, die Luftleitprofile 22, 24, 26, 28, 30 und den Luftführungs-Außenmantel 2 am Gehäuse 4 befestigt ist, ist er gehäusefest und der Ultraschallgeber 72 daher elastisch an das Gehäuse 4 gekoppelt, was die Schwingungsfähigkeit der Wirkstoff-Zuführungsdüse 66 verbessert. Damit kein Wirkstoff zu dem elastischen Ring 79 gelangt, umschließt ein ringkonischer Ansatz 81 an dem Körper 64 mit geringem Abstand den Radialflansch 74.

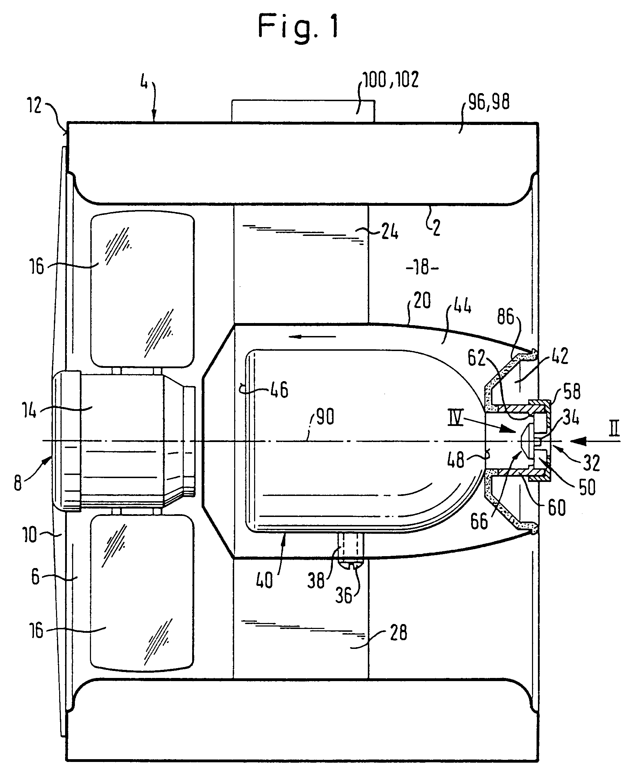

Wie aus Fig. 1 ersichtlich, ist der Ringeinlaß 42 durch einen Ringfilter 86 abgedeckt.

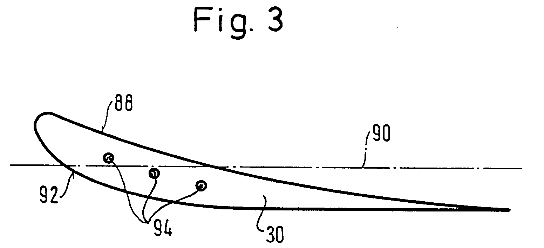

Wie aus Fig. 3 ersichtlich, ist in diesem Ausführungsbeispiel das Leitprofil 30 hohl und stromlinienförmig ausgebildet. Seine Innenfläche 88 hat eine Form, die den nicht hohlen Luftleitprofilen 22, 24, 26, 28 entspricht und in Radialsicht die gemeinsame Achse 90 des Ventilators 8 und des Kompressors 40 schneidet. Seine Außenfläche 92 ist stärker gewölbt. In dem Luftleitprofil 30 sind elektrische Leitungen 94 verlegt.

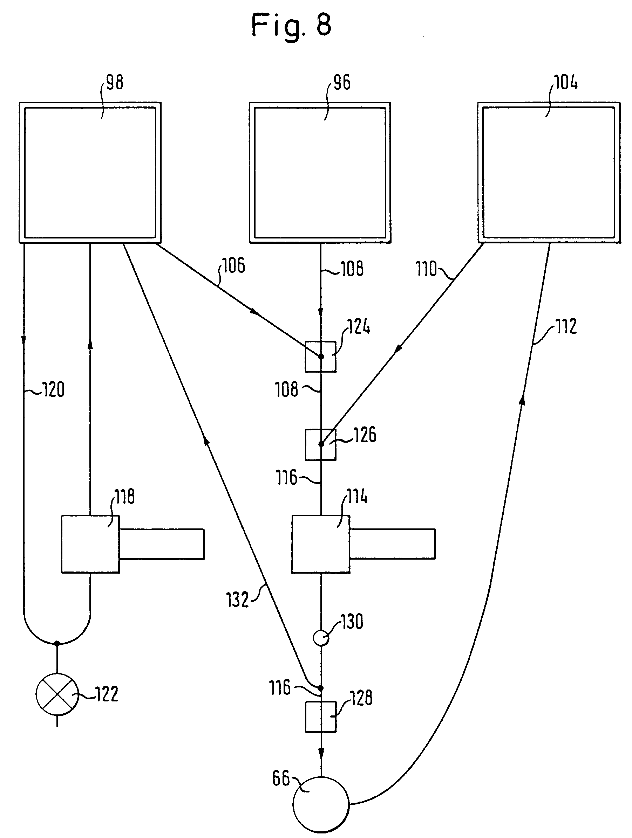

Von einem Spülmitteltank 96 und einem Wirkstofftank 98 im Gehäuse 4, die nach oben aus dem Gehäuse 4 herausragende Füllstutzen 100, 102 aufweisen, und gegebenenfalls von einem äußeren anzuschließenden dritten Tank 104 (siehe Fig. 8) sind Leitungen 106, 108, 110 zu einer gemeinsamen, eine Pumpe 114 enthaltenden Zuführungsleitung 116 zu der Wirkstoff-Zuführungsdüse 66 geführt. Dem Wirkstofftank 98 ist eine zweite Pumpe 118 in einer Umwälzleitung 120 zugeordnet, die einen Ablaßhahn 122 aufweist. Die Leitung 106 ist mit der Leitung 108 über ein 3-Wege-2-Stellungs-Ventil 124 verbunden, die Leitung 110 ist mit der Leitung 108 über ein 3-Wege-2-Stellungs-Ventil 126 verbunden. Beide diese Ventile 124, 126 liegen stromaufwärts der Pumpe 114. Zwischen der Pumpe 114 und der Wirkstoff-Zuführungsdüse 66 liegt in der gemeinsamen Zuführungsleitung 116 ein 2-Wege-2-Stellungs-Sperrventil 128. Zwischen dem Ventil 128 und der Pumpe 114 liegt in der Leitung 116 ein Ja/Nein-Durchflußmesser 130. Die Ventile 124, 126, 128 sind als Magnetventile ausgebildet und in eingangs beschriebener Weise steuerbar. Zwischen dem Ja/Nein-Durchflußmesser 130 und dem Ventil 128 ist von der Leitung 116 eine Leitung 132 zur Rückführung von Wirkstoff in den Wirkstofftank 98 abgezweigt. Von der Wirkstoff-Zuführungsdüse 66 führt eine Leitung 112 zu dem äußeren Tank 104, um diesen Tank 104 unter Druck zu setzen.

Die Tanks 96, 98 sind in einem Raum 134 zwischen dem Gehäuse 4 und dem Luftführungs-Außenmantel 2 oben symmetrisch zur vertikalen Längsmittelebene des Gehäuses 4 angeordnet. In diesem Raum 134 ist unten Platz für Elektroinstallationen.

## Patentansprüche

1. Vorrichtung zum Zerstäuben eines Wirkstoffs - insbesondere zur Schädlingsbekämpfung, zum Pflanzenschutz, zum Vorratsschutz, zur Desinfektion, zur Geruchsneutralisation oder zur Entkeimung - mit einem einen Luftführungs-Außenmantel (2) umschließenden Gehäuse (4), mit einem in einem Einlaßabschnitt (6) des Luftführungs-Außenmantels (2) angeordneten Ventilator (8), mit einem stromabwärts des Ventilators (8) angeordneten Luftführungs-Innenmantel (20), mit sich zwischen dem Luftführungs-Außenmantel (2) und dem Luftführungs-Innenmantel (20) erstreckenden Luftleitprofilen (22, 24, 26, 28, 30) und mit einer von dem Luftführungs-Innenmantel (20) umschlossenen Zerstäuberanordnung (32) zum Zerstäuben von ihr zugeführtem, aus einer stromabwärts gerichteten Wirkstoffaustrittsöffnung (34) im Luftführungs-Innenmantel (20) austretendem Wirkstoff,
**dadurch gekennzeichnet,**
daß sich in dem Luftführungs-Innenmantel (20) ein Kompressor (40) befindet, der Luft durch einen die Wirkstoffaustrittsöffnung (34) umschließenden Ringeinlaß (42) und einen zwischen dem Kompressor (40) und dem Luftführungs-Innenmantel (20) gebildeten Ringkanal (44) in seine dem Ventilator (8) zugewandte Einlaßöffnung (46) einsaugt und die von ihm komprimierte Luft durch seine der Wirkstoffaustrittsöffnung (34) zugewandte Auslaßöffnung (48) einer die Wirkstoffaustrittsöffnung (34) umschließenden Wirbelstromdüse (50) zuführt.

2. Vorrichtung nach Anspruch 1, dadurch gekennzeichnet, daß die Wirbelstromdüse (50) einen Kranz von unter gleichen Winkeln zu Radialen geneigten, einen zentralen Bereich (52) freilassenden Leitflächen (54, 56) aufweist, deren stromabwärtige Kanten radial außen durch eine Ringfläche abgedeckt sind.

3. Vorrichtung nach Anspruch 2, dadurch gekennzeichnet, daß die Leitflächen (54) eben sind.

4. Vorrichtung nach Anspruch 2, dadurch gekennzeichnet, daß die Leitflächen (56) gekrümmt sind.

5. Vorrichtung nach einem der Ansprüche 2 bis 4, dadurch gekennzeichnet, daß an die stromaufwärtigen Kanten der Wirbelstromdüse (50) ein Körper (64) einer Wirkstoff-Zuführungsdüse (66) angesetzt ist, die einen in den zentralen Bereich (52) der Wirbelstromdüse (50) ragenden Wirkstoff-Austrittsnippel (68) aufweist.

6. Vorrichtung nach Anspruch 5, dadurch gekennzeichnet, daß an die Wirkstoff-Zuführungsdüse (66) ein Ultraschallgeber (72) gekoppelt ist, der die Wirkstoff-Zuführungsdüse (66) in Axialschwingungen versetzt, und daß das Austrittsende des Wirkstoff-Austrittsnippels (68) der Wirkstoff-Zuführungsdüse (66) von einem Radialflansch (74) umschlossen ist.

7. Vorrichtung nach Anspruch 6, dadurch gekennzeichnet, daß als Ultraschallgeber (72) zwischen einem Halteflansch (76) der Wirkstoff-Zuführungsdüse (66) und einem Wirkstoffeinlaß (78) der Wirkstoff-Zuführungsdüse (66) ein ringförmiger Piezoerreger (80, 82) eingesetzt ist.

8. Vorrichtung nach Anspruch 7, dadurch gekennzeichnet, daß an die dem Wirkstoffeinlaß (78) zugewandte Seite des Piezoerregers (80, 82) ein Ringblock (84) angesetzt ist, der eine wesentlich größere Masse hat als der stromabwärts des Halteflansches (76) liegende Teil der Wirkstoff-Zuführungsdüse (66).

9. Vorrichtung nach einem der vorstehenden Ansprüche, dadurch gekennzeichnet, daß der Ringeinlaß (42) durch ein Ringfilter (86) abgedeckt ist.

10. Vorrichtung nach einem der vorstehenden Ansprüche, dadurch gekennzeichnet, daß wenigstens eines (30) der Luftleitprofile (22, 24, 26, 28, 30) hohl ist und elektrische Leitungen (94) zum Kompressor (40) und gegebenenfalls zum Ultraschallgeber (72) umschließt.

11. Vorrichtung nach einem der vorstehenden Ansprüche, dadurch gekennzeichnet, daß von einem Spülmitteltank (96) und einem Wirkstofftank (98) im Gehäuse (4) und gegebenenfalls von einem äußeren anzuschließenden dritten Tank (104) Leitungen (106, 108, 11) zu einer gemeinsamen, eine Pumpe (114) enthaltenden Zuführungsleitung (116) zu der Wirkstoff-Zuführungsdüse (66) führen.

12. Vorrichtung nach Anspruch 11, dadurch gekennzeichnet, daß dem Wirkstofftank (98) eine eine zweite Pumpe (118) enthaltende Umwälzleitung (120) zugeordnet ist.

13. Vorrichtung nach Anspruch 11 oder 12, dadurch gekennzeichnet, daß sich in den Leitungen (106, 108, 110, 116) von einer Programmsteuerung und einem Ja/Nein-Durchflußmesser (130) in der gemeinsamen Zuführungsleitung (130) steuerbare Magnetventile (124, 126, 128) befinden.

14. Vorrichtung nach einem der vorstehenden Ansprüche, dadurch gekennzeichnet, daß im Raum (134) zwischen dem Gehäuse (4) und dem Luftführungs-Außenmantel (2) oben symmetrisch zur vertikalen Längsmittelebene des Gehäuses (4) je ein Tank (96, 98) angeordnet ist und in diesem Raum (134) unten Elektroinstallationen vorgesehen sind.

15. Vorrichtung nach einem der vorstehenden Ansprüche, gekennzeichnet durch die Kombination eines Wirbelstromzerstäubers (50) und eines Ultraschallzerstäubers (72, 74).

## Claims

1. An apparatus for atomizing an active substance - in particular for pest control, crop protection, stock protection, disinfection, odor neutralization or degerminating - comprising:
a casing (4) surrounding an air-ducting outer shell (2),
a fan (8) arranged in an inlet section (6) of said air-ducting outer shell (2),
an air-ducting inner shell (20) arranged downstream of said fan (8),
air-deflecting profiles (22, 24, 26, 28, 30) extending between said air-ducting outer shell (2) and said air-ducting inner shell (20), and
an atomizer arrangement (32) surrounded by said air-ducting inner shell (20) for atomizing active substance fed to it, which active substance emerges atomized from a downstream-directed active-substance outlet (34) opening in said air-ducting inner shell (20),
**characterized in**
that a compressor (40) is located in said air-ducting inner shell (20), which compressor (40) sucks in air through an annular inlet (42) surrounding said active-substance outlet opening (34) and an annular channel (44) defined between said compresssor (40) and said air-ducting inner shell (20) into its inlet opening (46) facing said fan (8), and feeds the air compressed by it through its outlet opening (48), facing said active-substance cutlet opening (34) to a swirling stream nozzle (50) surrounding said active-substance outlet opening (34).

2. The apparaus as claimed in claim 1, characterized in that said swirling stream nozzle (50) has a ring of deflecting surfaces (54, 56) which are inclined at equal angles with respect to radials, and which leave a central region (52) free, the downstream edges of said deflecting surfaces (54, 56) being covered over radially on the outside by an annular face.

3. The apparatus as claimed in claim 2, characterized in that said deflecting surfaces (54) are plane.

4. The apparatus as claimed in claim 2, characterized in that said deflecting surfaces (56) are curved.

5. The apparatus as claimed in any of claims 2 to 4 characterized in that there is placed against the upstream edges of said swirling stream nozzle (50) a body (64) of an active-substance feed nozzle (66) which has an active-substance outlet nipple (68) protruding into the central region (52) of said swirling stream nozzle (50).

6. The apparatus as claimed in claim 5 characterized in that there is coupled to said active-substance feed nozzle (66) an ultrasonic generator (72) which sets said active-substance feed nozzle (66) into axial oscillations, and that the outlet end of the active-substance outlet nipple (68) of said active-substance feed nozzle (66) is surrounded by a radial flange (74).

7. The apparatus as claimed in claim 6 characterized in that the ultrasonic generator (72) includes an annular piezoelectric exciter (80, 82) fitted between a retaining flange (76) of said active-substance feed nozzle (66) and an active-substance inlet (78) of said active-substance feed nozzle (66).

8. The apparatus as claimed in claim 7 characterized in that there is fixed to the side of said piezoelectric exciter (80, 82) facing said active-substance inlet (78) an annular block (84) which has a substantially greater mass than the part of said active-substance feed nozzle (66) lying downstream of said retaining flange (76).

9. The apparatus as claimed in any of the preceding claims characterized in that said annular inlet (42) is covered over by an annular filter (86).

10. The apparatus as claimed in any of the preceding claims characterized in that at least one (30) of said air-deflecting profiles (22, 24, 26, 28, 30) is hollow and surrounds electrical lines (94) to said compressor (40) and - where appropriate - to said ultrasonic generator (72).

11. The apparatus as claimed in any of the preceding claims characterized in that lines (106, 108, 11) lead from a rinsing-agent tank (30) and an active-substance tank (98) in the casing (4) - and where appropriate - from an outer third tank (104) to be connected, to a common feed line (116) containing a pump (114), to said active-substance feed nozzle (66).

12. The apparatus as claimed in claim 11 characterized in that a circulating line (120) containing a second pump (118) is associated with said active-substance tank (98).

13. The apparatus as claimed in claim 11 or claim 12 characterized in that solenoid valves (124, 126, 128) are located in said lines (106, 108, 110, 116) which can be controlled by a program control and a yes/no flowmeter in said common feed line (130).

14. The apparatus as claimed in any of the preceding claims characterized in that each a tank (96, 98) is arranged in the space (134) between said casing (4) and said air-ducting outer shell (2) at the top symmetrically with respect to the vertical longitudinal center plane of said casing (4), and that electrical installations are provided in this space (134) at the bottom.

15. The apparatus as claimed in any of the preceding claims characterized by the combination of a swirling stream atomizer (50) and an ultrasonic atomizer (72, 74).

## Revendications

1. Dispositif de pulvérisation d'une substance active - notamment pour une lutte contre des parasites, la protection de plantes, la protection de stocks, une désinfection, une neutralisation d'odeurs ou une stérilisation - comprenant un boîtier (4) entourant une enveloppe externe de guidage d'air (2), un ventilateur (8) disposé dans une section d'entrée (6) de l'enveloppe externe de guidage d'air (20), une enveloppe interne de guidage d'air (2) disposée en aval du ventilateur (8) par rapport au courant, des éléments profilés déflecteurs d'air (22, 24, 26, 28, 30) s'étendant entre l'enveloppe externe de guidage d'air (2) et l'enveloppe interne de guidage d'air (20), et un agencement de pulvérisation (32) entouré par l'enveloppe interne de guidage d'air (20) pour pulvériser une substance active qui lui est amenée par une ouverture de sortie de substance active (34) dirigée vers l'aval par rapport au courant dans l'enveloppe interne de guidage d'air (20),
caractérisé en ce qu'il est prévu dans l'enveloppe interne de guidage d'air (20) un compresseur (40) qui aspire de l'air dans son ouverture d'entrée (46) qui est tournée vers le ventilateur (8) par une entrée annulaire (42) entourant l'ouverture de sortie de substance active (34) et une canalisation annulaire (44) formée entre le compresseur (40) et l'enveloppe interne de guidage d'air (20), et qui dirige l'air qu'il a comprimé par son ouverture de sortie (48) qui est tournée vers l'ouverture de sortie de substance active (34) vers une buse à courant tourbillonnaire (50) entourant l'ouverture de sortie de substance active (34).

2. Dispositif selon la revendication 1, caractérisé en ce que la buse à courant tourbillonnaire (50) comprend une couronne de surfaces déflectrices (54, 56) inclinées de façon égale en direction radiale et laissant libre une région centrale (52), surfaces dont les bords d'aval par rapport au courant sont recouverts radialement vers l'extérieur par une surface annulaire.

3. Dispositif selon la revendication 2, caractérisé en ce que les surfaces déflectrices (54) sont planes.

4. Dispositif selon la revendication 2, caractérisé en ce que les surfaces déflectrices (56) sont bombées.

5. Dispositif selon l'une quelconque des revendications 2 à 4, caractérisé en ce qu'un corps (64) de buse de distribution de substance active (66) est monté sur les bords de la buse à courant tourbillonnaire (50) qui sont en amont par rapport au courant, buse de distribution qui comprend un raccord de sortie de substance active (68) faisant saillie dans la zone centrale (52) de la buse à courant tourbillonnaire (50).

6. Dispositif selon la revendication 5, caractérisé en ce qu'un générateur d'ultrasons (72) est accouplé à la buse de distribution de substance active (66), qui imprime à la buse de distribution de substance active (66) des oscillations axiales, et en ce que l'extrémité de sortie du raccord de sortie de substance active (68) de la buse de distribution de substance active (66) est entourée par une bride radiale (74).

7. Dispositif selon la revendication 6, caractérisé en ce qu'on utilise en tant que générateur d'ultrasons (72) un excitateur piézoélectrique de forme annulaire (80, 82) monté entre une bride de retenue (76) de la buse de distribution de substance active (66) et une entrée de substance active (78) de la buse de distribution de substance active (66).

8. Dispositif selon la revendication 7, caractérisé en ce qu'un bloc annulaire (84) est monté sur le côté de l'excitateur piézoélectrique (80, 82) qui est tourné vers l'entrée à substance active (78), bloc qui présente une masse relativement plus importante que la partie de la buse de distribution de substance active (66) qui est située en aval de la bride de retenue (76) par rapport au courant.

9. Dispositif selon l'une quelconque des revendications précédentes, caractérisé en ce que l'entrée annulaire (42) est recouverte par un filtre annulaire (86).

10. Dispositif selon l'une quelconque des revendications précédentes, caractérisé en ce qu'au moins l'un (30) des éléments profilés déflecteurs d'air (22, 24, 26, 28, 30) est creux et contient des conducteurs électriques (94) allant au compresseur (40) et éventuellement au générateur d'ultrasons (72)

11. Dispositif selon l'une quelconque des revendications précédentes, caractérisé en ce que des conduites (106, 108, 11) s'étendent entre un réservoir à agent de rinçage (96) et un réservoir à substance active (98) contenus dans le boîtier (4) et éventuellement un troisième réservoir (104) extérieur et voisin et une conduite d'amenée commune (116) allant à la buse de distribution de substance active (66) et comprenant une pompe (114).

12. Dispositif selon la revendication 11, caractérisé en ce qu'une conduite de mise en circulation (111) contenant une seconde pompe (118) est associée au réservoir de substance active (98).

13. Dispositif selon la revendication 11 ou 12, caractérisé en ce que des électrovannes (124, 126, 128) pouvant être commandées par une commande programmée sont disposées dans les conduites (106, 108, 110, 116) et un débitmètre tout-ou-rien (130) dans la conduite d'amenée commune (116).

14. Dispositif selon l'une quelconque des revendications précédentes, caractérisé en ce que deux réservoirs (96, 98) sont disposés dans l'espace (134) compris entre le boîtier (4) et l'enveloppe externe de guidage d'air (2) et symétriquement à la partie supérieure par rapport au plan longitudinal médian vertical du boîtier (4), ainsi que des installations électriques dans la partie inférieure de cet espace (134).

15. Dispositif selon l'une quelconque des revendications précédentes, caractérisé par la combinaison d'une pulvérisateur à courant tourbillonnaire (50) et d'un pulvérisateur à ultrasons (72, 74).
